(19) Europäisches Patentamt
European Patent Office
Office européen des brevets

(11) Veröffentlichungsnummer: **0 043 923**
**B1**

# (12) EUROPÄISCHE PATENTSCHRIFT

(45) Veröffentlichungstag der Patentschrift: **13.02.85**

(51) Int. Cl.⁴: **C 07 D 405/06**, C 07 D 405/14, C 07 D 409/14, A 01 N 43/64

(21) Anmeldenummer: **81104593.9**

(22) Anmeldetag: **15.06.81**

(54) **1,3-Dioxan-5-yl-alkenyltriazole, ihre Herstellung, ihre Verwendung zur Regulierung des Pflanzenwachstums und Mittel dafür.**

(30) Priorität: **10.07.80 DE 3026140**

(43) Veröffentlichungstag der Anmeldung: **20.01.82 Patentblatt 82/3**

(45) Bekanntmachung des Hinweises auf die Patenterteilung: **13.02.85 Patentblatt 85/7**

(84) Benannte Vertragsstaaten: **AT BE CH DE FR GB IT LI LU NL SE**

(56) Entgegenhaltungen:
**DE - A - 2 739 352**
**FR - A - 2 290 898**
**FR - A - 2 434 164**
**FR - A - 2 440 367**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Feuerherd, Karl-Heinz, Dr., Berner Weg 34, D-6700 Ludwigshafen (DE)**
Erfinder: **Zeeh, Bernd, Dr., Thorwaldsenstrasse 5, D-6700 Ludwigshafen (DE)**
Erfinder: **Jung, Johann, Dr., Hardenburgstrasse 19, D-6703 Limburgerhof (DE)**

**EP 0 043 923 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue 1,3-Dioxan-5-yl-al-kenyltriazole, Verfahren zu ihrer Herstellung, Verfahren zur Regulierung des Pflanzenwachstums mit diesen Verbindungen und Mittel zur Regulierung des Pflanzenwachstums, die diese Verbindungen enthalten.

Es ist bekannt, 2-Chlorethyl-trimethylammoniumchlorid (Chlorcholinchlorid, CCC) (vgl. US-PS 3 156 554) zur Beeinflußung des Pflanzenwachstums zu verwenden. Mit seiner Hilfe läßt sich z. B. eine Hemmung des Längenwachstums bei einigen Getreidearten und eine Hemmung des vegetativen Wachstums bei einigen anderen Kulturpflanzen erreichen. Die Wirkung dieser Substanz ist jedoch, insbesondere bei niedrigen Aufwandmengen, nicht immer ausreichend und genügt den Anforderungen der Praxis nicht.

Es ist ferner bekannt, 3,3-Dimethyl-2-(1,2,4-triazolyl-(1))-1-(4-chlorbenzoyl)-butan zur Beeinflußung des Pflanzenwachstums zu verwenden (DE-OS 2 739 352).

Dioxolanyltriazole sind schon aus der FR-A 2 290 898 bekannt, wobei sowohl eine fungizide als auch eine wachstumsregulierende Wirkung angegeben wird.

Soweit die Mittel Der FR-A 2 290 898 sich praktisch durchgesetzt haben, sind sie allerdings nur als Fungizide von Interesse; die wachstumsregulierende Wirkung ist offenbar gering und jedenfalls nicht im erforderlichen Maße vorhanden, so daß die Bereitstellung chemisch ähnlicher Mittel mit ausreichend wachstumsregulierender Wirkung wünschenswert erscheint.

Es wurde nun gefunden, daß 1,3-Dioxan-5-yl-alkenyltriazole der Formel I

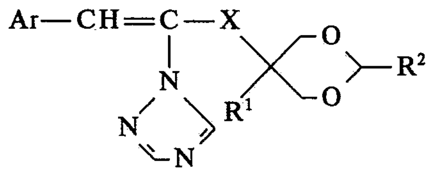

(I)

in der

$R^1$ und $R^2$ gleich oder verschieden sind und Wasserstoff oder Alkyl mit 1 bis 5 C-Atomen bedeuten,

Ar Furanyl, Thienyl, Biphenylyl, Naphthyl oder Phenyl bedeutet, wobei der Phenylrest durch Alkyl, Alkoxy oder Alkenyl mit jeweils 1 bis 5 C-Atomen, Fluor, Chlor, Brom, Iod, Nitro, Trifluormethyl und ferner durch Phenoxy substituiert sein kann und

X eine C=O, $-CH(OH)-$ oder $-CH(O-COR^3)$-Gruppe bedeutet, wobei $R^3$ einen gegebenenfalls durch Halogen oder Alkoxy substituierten Alkyl mit 1 bis 5 C-Atomen oder Alkenyl mit 2 bis 5 C-Atomen oder Aryl bedeutet,

sowie deren Salze und Metallkomplexe, hervorragend zur Beeinflußung des Pflanzenwachstums geeignet sind und eine sehr gute Pflanzenverträglichkeit besitzen.

Die erfindungsgemäßen Verbindungen können in zwei geometrischen Isomerenformen (Z und E) entstehen, je nach der Anordnung der Substituenten an der Doppelbindung. Die Verbindungen der Formel I können zusätzlich als Enantiomerengemische, bzw. als Racemate vorliegen, wenn X ein Chiralitätszentrum darstellt. Aus den bei der Synthese üblicherweise anfallenden Isomerengemischen kann man mit bekannten Methoden einheitliche geometrische Isomere bzw. einheitliche Enantiomere erhalten. Als Mittel zur Beeinflußung des Pflanzenwachstums kann man sowohl die einheitlichen geometrischen Isomeren bzw. Enantiomeren wie auch deren bei der Synthese anfallenden Gemische verwenden. Bevorzugt werden die letzteren verwendet.

$R^1$ und $R^2$ bedeuten beispielsweise Wasserstoff, Methyl, Ethyl, n-Propyl, Isopropyl, n-Butyl, sek.-Butyl, iso-Butyl, tert.-Butyl, n-Pentyl und Neopentyl.

Ar bedeutet beispielsweise 2-Furanyl, 2- und 3-Thienyl, 4-Biphenyl, 1- und 2-Naphthyl, Phenyl, 4-Fluorphenyl, 2-Chlorphenyl, 3-Chlorphenyl, 4-Chlorphenyl, 4-Bromphenyl, 2,4-Dichlorphenyl, 3,4-Dichlorphenyl, 3,5-Dichlor-2-methoxyphenyl, 2,3,4-Trichlorphenyl, 2-Methoxyphenyl, 2,4-Dimethoxyphenyl, 3-Methoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 3-Nitrophenyl, 4-Nitrophenyl, 3-Trifluormethylphenyl, 4-Trifluormethylphenyl und 4-Phenoxyphenyl.

$R^3$ bedeutet beispielsweise Methyl, Ethyl, n-Propyl, Isopropyl, Chlormethyl, Chlorpropyl, Methoxymethyl, Vinyl und Propenyl.

Als Beispiele für die neuen Verbindungen seien folgende genannt:

1-(5-Methyl-1,3-dioxan-5-yl)-2-(1,2,4-triazolyl-(1))-3-phenyl-propen-1-on,
1-(5-Methyl-1,3-dioxan-5-yl)-2-(1,2,4-triazolyl-(1))-3-phenyl-propen-1-ol,
1-(2-Ethyl-5-methyl-1,3-dioxan-5-yl)-2-(1,2,4-triazolyl-(1))-3-phenyl-propen-1-ol,
1-(2-Ethyl-5-methyl-1,3-dioxan-5-yl)-2-(1,2,4-triazolyl-(1))-3-phenyl-propen-1-on,
1-(2-n-Propyl-5-methyl-1,3-dioxan-5-yl)-2-(1,2,4-triazolyl-(1))-3-phenyl-propen-1-on,

1-(2-n-Propyl-5-methyl-1,3-dioxan-5-yl)-2-(1,2,4-triazolyl-(1))-3-phenyl-propen-1-ol,
1-(5-Methyl-1,3-dioxan-5-yl)-1-acetoxy-(1,2,4-triazolyl-(1))-3-phenyl-propen-2-,
1-(5-Methyl-1,3-dioxan-5-yl)-1-propionyloxy-(1,2,4-triazolyl-(1))-3-phenyl-propen-2,
1-(2-Ethyl-5-methyl-1,3-dioxan-5-yl)-1-propionyloxy-(1,2,4-triazolyl-(1))-3-phenyl-propen-2,
1-(2-Ethyl-5-methyl-1,3-dioxan-5-yl)-2-benzoloxy-(1,2,4-triazolyl-(1))-3-phenyl-propen-2,
1-(2-Ethyl-5-methyl-1,3-dioxan-5-yl)-2-chloracetoxy-(1,2,4-triazolyl-(1))-3-phenyl-propen-2,
1-(5-Methyl-1,3-dioxan-5-yl)-2-(1,2,4-triazolyl-(1))-3-(2-furanyl)-propen-1-on,
1-(5-Methyl-1,3-dioxan-5-yl)-2-(1,2,4-triazolyl-(1))-3-(2-furanyl)-propen-1-ol,
1-(5-Methyl-1,3-dioxan-5-yl)-1-acetoxy-2-(1,2,4-triazolyl-(1))-3-2-furanyl)-propen-2,
1-(5-Methyl-1,3-dioxan-5-yl)-2-(1,2,4-triazolyl-(1))-3-(1-naphthyl)-propen-1-on,
1-(5-Methyl-1,3-dioxan-5-yl)-2-(1,2,4-triazolyl-(1))-3-(1-naphthyl)-propen-1-ol,
1-(2-Ethyl-5-methyl-1,3-dioxan-5-yl)-2-(1,2,4-triazolyl-(1))-3-(1-naphthyl)-propen-1-on,
1-(2-Ethyl-5-methyl-1,3-dioxan-5-yl)-2-(1,2,4-triazolyl-(1))-3-(1-naphthyl)-propen-1-ol,
1-(2,5-Diethyl-1,3-dioxan-5-yl)-2-(1,2,4-triazolyl-(1))-3-(1-naphthyl)-propen-1-on,
1-(2,5-Diethyl-1,3-dioxan-5-yl)-2-(1,2,4-triazolyl)-(1))-3-(1-naphthyl)-propen-1-ol,
1-(2-Ethyl-5-methyl-1,3-dioxan-5-yl)-1-acetoxy-(1,2,4-triazolyl-(1))-3-(1-naphthyl)-propen-2,
1-(5-Methyl-1,3-dioxan-5-yl)-2-(1,2,4-triazolyl-(1))-3-(4-biphenylyl)-propen-1-on,
1-(5-Methyl-1,3-dioxan-5-yl)-2-(1,2,4-triazolyl-(1))-3-(4-biphenylyl)-propen-1-ol,
1-(5-Methyl-1,3-dioxan-5-yl)-2-(1,2,4-triazolyl-(1))-3-(4-fluorphenyl)-propen-1-on,
1-(5-Methyl-1,3-dioxan-5-yl)-2-(1,2,4-triazolyl-(1))-3-(4-fluorphenyl)-propen-1-ol,
1-(5-Methyl-1,3-dioxan-5-yl)-1-propionyloxy-2-(1,2,4-triazolyl-(1))-3-(4-fluorphenyl)-propen-2,
1-(5-Methyl-1,3-dioxan-5-yl)-2-(1,2,4-triazolyl-(1))-3-(2-chlorphenyl)-propen-1-on,
1-(5-Methyl-1,3-dioxan-5-yl)-2-(1,2,4-triazolyl-(1))-2-chlorphenyl)-propen-1-ol,
1-(2-Ethyl-5-methyl-1,3-dioxan-5-yl)-2-(1,2,4-triazolyl-(1))-3-(2-chlorphenyl)-propen-1-on,
1-(2-Ethyl-5-methyl-1,3-dioxan-5-yl)-2-(1,2,4-triazolyl-(1))-3-(2-chlorphenyl)-propen-1-ol,
1-(2-Ethyl-5-methyl-1,3-dioxan-5-yl)-1-acetoxy-(1,2,4-triazolyl-(1))-3-(2-chlorphenyl)-propen-2,
1-(5-Methyl-1,3-dioxan-5-yl)-2-(1,2,4-triazolyl-(1))-3-(3-chlorphenyl)-propen-1-on,
1-(5-Methyl-1,3-dioxan-5-yl)-2-(1,2,4-triazolyl-(1))-3-(3-chlorphenyl)-propen-1-ol,
1-(5-Methyl-1,3-dioxan-5-yl)-1-propionyloxy-2-(1,2,4-triazolyl-(1))-3-(3-chlorphenyl)-propen-2,
1-(5-Methyl-1,3-dioxan-5-yl)-2-(1,2,4-triazolyl-(1))-3-(4-chlorphenyl)-propen-1-on,
1-(5-Methyl-1,3-dioxan-5-yl)-2-(1,2,4-triazolyl-(1))-3-(4-chlorphenyl)-propen-1-ol,
1-(2,5-Dimethyl-1,3-dioxan-5-yl)-2-(1,2,4-triazolyl-(1))-3-(4-chlorphenyl)-propen-1-on,
1-(2,5-Dimethyl-1,3-dioxan-5-yl)-2-(1,2,4-triazolyl-(1))-3-(4-chlorphenyl)-propen-1-ol,
1-(2-Ethyl-5-methyl-1,3-dioxan-5-yl)-2-(1,2,4-triazolyl-(1))-(3-(4-chlorphenyl)-propen-1-on,
1-(2-Ethyl-5-methyl-1,3-dioxan-5-yl)-2-(1,2,4-triazolyl-(1))-3-(4-chlorphenyl)-propen-1-ol,
1-(2-n-Propyl-5-methyl-1,3-dioxan-5-yl)-2-(1,2,4-triazolyl-(1))-3-(4-chlorphenyl)-propen-1-on,
1-(2-n-Propyl-5-methyl-1,3-dioxan-5-yl)-2-(1,2,4-triazolyl-(1))-3-(4-chlorphenyl)-propen-1-ol,
1-(2,5-Diethyl-1,3-dioxan-5-yl)-2-(1,2,4-triazolyl-(1))-3-(4-chlorphenyl)-propen-1-ol,
1-(2,5-Diethyl-1,3-dioxan-5-yl)-2-(1,2,4-triazolyl-(1))-3-(4-chlorphenyl)-propen-1-on,
1-(2-(Ethyl-5-methyl-1,3-dioxan-5-yl)-1-acetoxy-2-(1,2,4-triazolyl-(1))-3-(4-chlorphenyl)-propen-2,
1-(2-Ethyl-5-methyl-1,3-dioxan-5-yl)-1-propionyloxy-2-(1,2,4-triazolyl-(1))-3-(4-chlorphenyl)-propen-2,
1-(2-Ethyl-5-methyl-1,3-dioxan-5-yl)-1-dichloracetoxy-2-(1,2,4-triazolyl-(1))-3-(4-chlorphenyl)-propen-2,
1-(5-Methyl-1,3-dioxan-5-yl)-2-(1,2,4-triazolyl-(1))-3-(4-bromphenyl)-propen-1-on,
1-(5-Methyl-1,3-dioxan-5-yl)-2-(1,2,4-triazolyl-(1))-3-(4-bromphenyl)-propen-1-ol,
1-(2-Ethyl-5-methyl-1,3-dioxan-5-yl)-2-(1,2,4-triazolyl-(1))-3-(4-bromphenyl)-propen-1-on,
1-(2-Ethyl-5-methyl-1,3-dioxan-5-yl)-2-(1,2,4-triazolyl-(1))-3-(4-bromphenyl)-propen-1-ol,
1-(2-n-Propyl-5-methyl-1,3-dioxan-5-yl)-2-(1,2,4-triazolyl-(1))-3-(4-bromphenyl)-propen-1-on,
1-(2-n-Propyl-5-methyl-1,3-dioxan-5-yl)-2-(1,2,4-triazolyl-(1))-3-(4-bromphenyl)-propen-1-ol,
1-(2,5-Diethyl-1,3-dioxan-5-yl)-2-(1,2,4-triazolyl-(1))-3-(4-bromphenyl)-propen-1-ol,
1-(2,5-Diethyl-1,3-dioxan-5-yl)-2-(1,2,4-triazolyl-(1))-3-(4-bromphenyl)-propen-1-on,
1-(2-Ethyl-5-methyl-1,3-dioxan-5-yl)-1-acetoxy-2-(1,2,4-triazolyl-(1))-3-(4-bromphenyl)-propen-2,
1-(2-Ethyl-5-methyl-1,3-dioxan-5-yl)-1-propionyloxy-2-(1,2,4-triazolyl-(1))-3-(4-bromphenyl)-propen-2,
1-(5-Methyl-1,3-dioxan-5-yl)-2-(1,2,4-triazolyl-(1))-3-(3,4-dichlorphenyl)-propen-1-on,
1-(5-Methyl-1,3-dioxan-5-yl)-2-(1,2,4-triazolyl-(1))-3-(3,4-dichlorphenyl)-propen-1-ol,

1-(2-Ethyl-5-methyl-1,3-dioxan-5-yl)-2-(1,2,4-triazolyl-(1))-3-(3,4-dichlorphenyl)-propen-1-on,
1-(2-Ethyl-5-methyl-1,3-dioxan-5-yl)-2-(1,2,4-triazolyl-(1))-3-(3,4-dichlorphenyl)-propen-1-ol,
1-(5-Methyl-1,3-dioxan-5-yl)-1-acetoxy-2-(1,2,4-triazolyl-(1))-3-(3,4-dichlorphenyl)-propen-2,
1-(2-Ethyl-5-methyl-1,3-dioxan-5-yl)-1-acetoxy-2-(1,2,4-triazolyl-(1))-3-(3,4-dichlorphenyl)-propen-2,
1-(5-Methyl-1,3-dioxan-5-yl)-2-(1,2,4-triazolyl)-(1))-3-(2,4-dichlorphenyl)-propen-1-on,
1-(5-Methyl-1,3-dioxan-5-yl)-2-(1,2,4-triazolyl-(1))-3-(2,4-dichlorphenyl)-propen-1-ol,
1-(2-Ethyl-5-methyl-1,3-dioxan-5-yl)-2-(1,2,4-triazolyl-(1))-3-(2,4-dichlorphenyl)-propen-1-on,
1-(2-Ethyl-5-methyl-1,3-dioxan-5-yl)-2-(1,2,4-triazolyl-(1))-3-(2,4-dichlorphenyl)-propen-1-ol,
1-(2,5-Diethyl-1,3-dioxan-5-yl)-2-(1,2,4-triazolyl-(1))-3-(2,4-dichlorphenyl)-propen-1-on,
1-(2,5-Diethyl-1,3-dioxan-5-yl)-2-(1,2,4-triazolyl-(1))-3-(2,4-dichlorphenyl)-propen-1-ol,
1-(2-Isopropyl-5-methyl-1,3-dioxan-5-yl)-2-(1,2,4-triazolyl-(1))-3-(2,4-dichlorphenyl)-propen-1-on,
1-(2-Isopropyl-5-methyl-1,3-dioxan-5-yl)-2-(1,2,4-triazolyl-(1))-3-(2,4-dichlorphenyl)-propen-1-ol,
1-(2-n-Butyl-5-methyl-1,3-dioxan-5-yl)-2-(1,2,4-triazolyl-(1))-3-(2,4-dichlorphenyl)-propen-1-on,
1-(2-n-Butyl-5-methyl-1,3-dioxan-5-yl)-2-(1,2,4-triazolyl-(1))-3-(2,4-dichlorphenyl)-propen-1-ol,
1-(5-Methyl-1,3-dioxan-5-yl)-1-acetoxy-2-(1,2,4-triazolyl-(1))-3-(2,4-dichlorphenyl)-propen-2,
1-(5-Methyl-1,3-dioxan-5-yl)-1-butyryloxy-2-(1,2,4-triazolyl-(1))-3-(2,4-dichlorphenyl)-propen-2,
1-(2-Ethyl-5-methyl-1,3-dioxan-5-yl)-1-acetoxy-2-(1,2,4-triazolyl-(1))-3-(2,4-dichlorphenyl)-propen-2,
1-(2-Ethyl-5-methyl-1,3-dioxan-5-yl)-1-propionyloxy-2-(1,2,4-triazolyl-(1))-3-(2,4-dichlorphenyl)-propen-2,
1-(5-Methyl-1,3-dioxan-5-yl)-2-(1,2,4-triazolyl-(1))-3-(2,3,4-trichlorphenyl)-propen-1-on,
1-(5-Methyl-1,3-dioxan-5-yl)-2-(1,2,4-triazolyl-(1))-3-(2,3,4-trichlorphenyl)-propen-1-ol,
1-(5-Methyl-1,3-dioxan-5-yl)-1-acetoxy-(1,2,4-triazolyl-(1))-3-(2,3,4-trichlorphenyl)-propen-2,
1-(2-Ethyl-5-methyl-1,3-dioxan-5-yl)-2-(1,2,4-triazolyl-(1))-3-(2,3,4-trichlorphenyl)-propen-1-on,
1-(2-Ethyl-5-methyl-1,3-dioxan-5-yl)-2-(1,2,4-triazolyl-(1))-3-(2,3,4-trichlorphenyl)-propen-1-ol,
1-(5-Methyl-1,3-dioxan-5-yl)-2-(1,2,4-triazolyl-(1))-3-(3-trifluormethylphenyl)-propen-1-on,
1-(5-Methyl-1,3-dioxan-5-yl)-2-(1,2,4-triazolyl-(1))-3-(3-trifluormethylphenyl)-propen-1-ol,
1-(5-Methyl-1,3-dioxan-5-yl)-1-propionyloxy-2-(1,2,4-triazolyl-(1))-3-(3-trifluormethylphenyl)-propen-2,
1-(5-Methyl-1,3-dioxan-5-yl)-2-(1,2,4-triazolyl-(1))-3-(4-trifluormethylphenyl)-propen-1-on,
1-(5-Methyl-1,3-dioxan-5-yl)-2-(1,2,4-triazolyl-(1))-3-(4-trifluormethylphenyl)-propen-1-ol,
1-(2-Ethyl-5-methyl-1,3-dioxan-5-yl)-2-(1,2,4-triazolyl-(1))-3-(4-trifluormethylphenyl)-propen-1-on,
1-(2-Ethyl-5-methyl-1,3-dioxan-5-yl)-2-(1,2,4-triazolyl-(1))-3-(4-trifluormethylphenyl)-propen-1-ol,
1-(5-methyl-1,3-dioxan-5-yl)-2-(1,2,4-triazolyl-(1))-3-(4-methylphenyl)-propen-1-on,
1-(5-Methyl-1,3-dioxan-5-yl)-2-(1,2,4-triazolyl-(1))-3-(4-methylphenyl)-propen-1-ol,
1-(2-Ethyl-5-methyl-1,3-dioxan-5-yl)-2-(1,2,4-triazolyl-(1))-3-(4-methylphenyl)-propen-1-on,
1-(2-Ethyl-5-methyl-1,3-dioxan-5-yl)-2-(1,2,4-triazolyl-(1))-3-(4-methylphenyl)-propen-1-ol,
1-(5-Methyl-1,3-dioxan-5-yl)-2-(1,2,4-triazolyl-(1))-3-(4-ethylphenyl)-propen-1-on,
1-(5-Methyl-1,3-dioxan-5-yl)-2-1,2,4-triazolyl-(1))-3-(4-ethylphenyl)-propen-1-ol,
1-(5-Methyl-1,3-dioxan-5-yl)-2-(1,2,4-triazolyl-(1))-3-(4-tert.-butylphenyl)-propen-1-on,
1-(5-Methyl-1,3-dioxan-5-yl)-2-(1,2,4-triazolyl-(1))-3-(4-tert.-butylphenyl)-propen-1-ol,
1-(5-Methyl-1,3-dioxan-5-yl)-2-(1,2,4-triazolyl-(1))-3-(4-methoxyphenyl)-propen-1-ol,
1-(5-Methyl-1,3-dioxan-5-yl)-2-(1,2,4-triazolyl-(1))-3-(4-methoxyphenyl)-propen-1-on,
1-(2-Ethyl-5-methyl-1,3-dioxan-5-yl)-2-(1,2,4-triazolyl-(1))-3-(4-methoxyphenyl)-propen-1-on,
1-(2-Ethyl-5-methyl-1,3-dioxan-5-yl)-2-(1,2,4-triazolyl-(1))-3-(4-methoxyphenyl)-propen-1-ol,
1-(5-Methyl-1,3-dioxan-5-yl)-2-(1,2,4-triazolyl-(1))-3-(4-ethoxyphenyl)-propen-1-on,
1-(5-Methyl-1,3-dioxan-5-yl)-2-(1,2,4-triazolyl-(1))-3-(4-ethoxyphenyl)-propen-1-ol,

1-(5-(Methyl-1,3-dioxan-5-yl)-2-(1,2,4-triazolyl-(1))-3-(4-n-butoxyphenyl)-propen-1-on,
1-(5-Methyl-1,3-dioxan-5-yl)-2-(1,2,4-triazolyl-(1))-3-(4-n-butoxyphenyl)-propen-1-ol,
1-(5-Methyl-1,3-dioxan-5-yl)-2-(1,2,4-triazolyl-(1))-3-(4-tert.-butoxyphenyl)-propen-1-ol,
1-(5-Methyl-1,3-dioxan-5-yl)-2-(1,2,4-triazolyl-(1))-3-(4-tert.-butoxyphenyl)-propen-1-on,
1-(5-Methyl-1,3-dioxan-5-yl)-2-(1,2,4-triazolyl-(1))-3-(4-nitrophenyl)-propen-1-on,
1-(5-Methyl-1,3-dioxan-5-yl)-2-(1,2,4-triazolyl-(1))-3-(4-nitrophenyl)-propen-1-ol.

Die Verbindungen der Formel I lassen sich herstellen, indem man einen Aldehyd der Formel II

$$Ar-CH=O \qquad (II)$$

in der Ar die oben genannten Bedeutung hat, mit einem 1-(1,3-Dioxan-5-yl)-2-(1,2,4-triazolyl-(1))-ethan-1-on-Derivat der Formel III

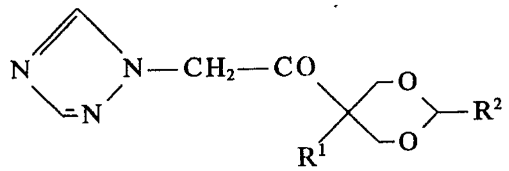

(III)

in welcher $R^1$ und $R^2$ die oben angegebenen Bedeutungen haben, kondensiert, ggf. die so erhaltenen Verbindungen zum Alkohol reduziert, ggf. die reduzierte Verbindung mit einem Säurehalogenid der Formel IV

$$Y-CO-R^3 \qquad (IV)$$

in der $R^3$ dasselbe wie oben bedeutet und Y Chlor oder Brom bedeutet, oder mit einem Säureanhydrid der Formel V

$$(R^3-CO)_2O \qquad (V)$$

verestert und die erhaltenen Verbindungen gegebenenfalls in ihre Salze oder Metallkomplexe überführt.

Die Verbindungen II werden mit den Verbindungen III gegebenenfalls in Gegenwart eines Lösungs- oder Verdünnungsmittels und gegebenenfalls unter Zusatz einer anorganischen oder organischen Base und/oder Säure bei Temperaturen zwischen −10 und 150°C gegebenenfalls unter azeotropischer Auskreisung des Reaktionswassers kondensiert. Zu den bevorzugten Lösungs- und Verdünnungsmitteln gehören Wasser, Alkohole wie Methanol, Ethanol, Isopropanol und tert.-Butanol, Kohlenwasserstoffe wie n-Hexan, n-Heptan, n- und iso-Octan, Cyclohexan, Tetrahydronaphthalin, Decahydronaphthalin, Toluol, Xylol, Cumol; Amide wie Dimethylformamid, Dimethylacetamid, N-Methylpyrrolidon und Ether wie Diethylether, Methyl-tert.-butylether, Dioxan, Tetrahydrofuran oder entsprechende Gemische.

Geeignete Basen und Säuren, die gegebenenfalls in katalytischen bis zu stöchiometrischen Mengen verwendet werden können, sind beispielsweise Alkali- und Erdalkalihydroxide, wie Natrium-, Kalium-, Calcium- und Bariumhydroxid, Alkoxide wie Natrium- und Kaliummethoxid, -ethoxid, -isopropoxid und tert.-butoxid, Acetate wie Natrium- oder Kaliumacetat, Amine wie Pyrrolidin, Piperidin, Triethylamin, N,N-Dimethylcyclohexylamin, ferner anorganische Säuren wie Salzsäure, Phosphorsäure und Schwefelsäure oder organische Säuren wie Ameisensäure, Essigsäure, p-Toluolsulfonsäure oder Trifluormethylsulfonsäure.

Die Umsetzungen werden im allgemeinen bei Temperaturen zwischen 20 und 150°C, drucklos oder unter Druck, kontinuierlich oder diskontinuierlich durchgeführt.

Die 1-(1,3-Dioxan-5-yl)-2-(1,2,4-triazolyl-(1))-ethan-1-one der Formel III sind neu. Sie können beispielsweise durch Umsetzung von 1-(1,3-Dioxan-5-yl)-2-halogen-ethan-1-onen der Formel VI

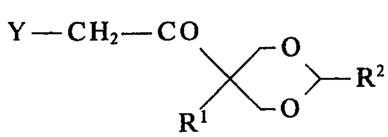

(VI)

in welcher $R^1$ und $R^2$ die oben angegebenen Bedeutungen haben und Y Chlor oder Brom bedeutet, mit dem Natriumsalz des 1,2,4-Triazols erhalten werden.

Die Verbindungen der Formel VI sind ebenfalls neu. Man erhält sie z. B. durch Umsetzung von bekannten (1,3-Dioxan-5-yl)-ethan-1-onen der Formel VII

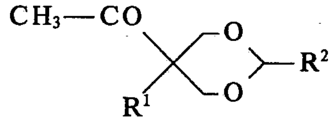

(VII)

in welcher $R^1$ und $R^2$ die oben angegebenen Bedeutungen haben mit Sulfurylchlorid nach D. P. Wyman und P. R. Kaufman, J. Org. Chem. 29, 1956 (1964) oder mit Brom in Formamid nach H. Bredereck und Mitarb., Chem. Ber. 93, 2083 (1960).

Die Reduktion der Ketone der Formel

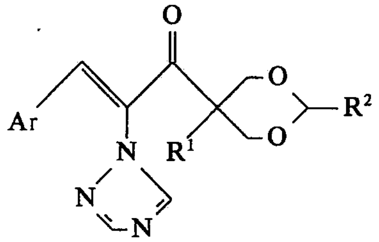

(I, X = CO)

in welcher Ar, $R^1$ und $R^2$ die oben genannten Bedeutungen haben, kann gegebenenfalls in Gegenwart eines Lösungs- oder Verdünnungsmittels, bei Temperaturen zwischen −20 bis 150°C zu Alkoholen der Formel Ib

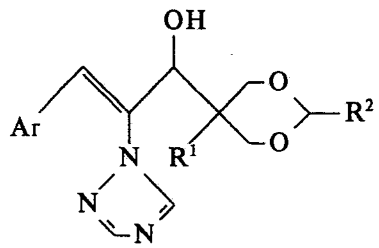

(I, X = CHOH)

in welcher Ar, $R^1$ und $R^2$ die oben angegebenen Bedeutungen haben, vorgenommen werden. Die Reduktion selbst kann mit Wasserstoff in Gegenwart eines Katalysators, mit komplexen Bor- bzw. Aluminiumhydriden, mit Aluminiumisopropoxid und Isopropanol, mit Natriumdithionit oder elektrochemisch erfolgen.

Geeignete Lösungs- oder Verdünnungsmittel hierfür sind beispielsweise Wasser, Methanol, Ethanol, Propanol, Isopropanol, Essigsäure, Diethylether, Tetrahydrofuran, Dioxan, Essigester, Toluol, Dimethylformamid oder entsprechende Gemische.

Zur katalytischen Hydrierung verwendet man Platin- oder Paladiumkatalysatoren auf inerten Trägern und hydriert bei Drucken von 2 bis 80 bar bis keine Wasserstoffaufnahme mehr erfolgt. Für die als Reduktionsmittel verwendbaren Hydride seien beispielsweise Natriumborhydrid oder Lithiumaluminiumhydrid genannt.

Die Veresterung der Alkohole der Formel I (X = CHOH) erfolgt gegebenenfalls in Gegenwart eines Lösungs- oder Verdünnungsmittels, gegebenenfalls unter Zusatz eines säurebindenden Mittels und gegebenenfalls unter Zusatz eines Reaktionsbeschleunigers. Hierzu eignen sich folgende Lösungsmittel: Diethylether, Tetrahydrofuran, Dioxan, n-Pentan, n-Hexan, Petrolether, Cyclohexan, Toluol, Methylenchlorid, Chloroform, Aceton, Methylethylketon, Acetonitril, Dimethylformamid, Acetanhydrid und Propionanhydrid.

Als säurebindende Mittel seien Basen genannt, beispielsweise Alkali- und Erdalkalicarbonate wie Natriumcarbonat, Kaliumcarbonat, Natriumhydrogencarbonat, Kaliumhydrogencarbonat, Calciumcarbonat, Bariumcarbonat, tertiäre Amine wie Triethylamin, N,N-Dimethylcyclohexylamin, N,N-Dimethylanilin, N-Methylpiperidin oder Pyridin.

Geeignete Reaktionsbeschleuniger sind Azole, wie 1,2,4-Triazol und Imidazol oder tertiäre Amine wie 4-Dimethylaminopyridin und 4-Pyrrolidinopyridin.

Die so erhaltenen Verbindungen der Formel I werden nach üblichen Methoden isoliert, ggf. gereinigt und ggf. mit Säuren oder Metallsalzen zu Salzen bzw. zu Metall-Komplexen umgesetzt.

Die folgenden Beispiele erläutern die Erfindung.

## Beispiel 1

### a) Herstellung des Ausgangsproduktes

Zu einer Lösung von 144 g (1 Mol) 5-Acetyl-5-methyl-1,3-dioxan und 85,5 g (1 Mol) Pyrrolidon in 500 ml Tetrahydrofuran wird in 2 Stunden bei 50°C die Lösung von 498 g (1 Mol) Pyrrolidon-Brom-Komplex in 1 l Tetrahydrofuran zugetropft. Nach 8stündigem Nachrühren bei 50°C wird der weiße Niederschlag von Pyrrolidon-Hydrobromid abgesaugt, mit 50 ml Tetrahydrofuran gewaschen und das Filtrat im Vakuum eingeengt. Man erhält 220 g (99%) rohes öliges 1-(5-Methyl-1,3-dioxan-5-yl)-2-brom-ethan-1-on.
Zu einer unter reinem Stickstoff gerührten Suspension von 100 g (1,1 Mol) Natrium-1,2,4-triazolid in 300 ml trockenem Tetrahydrofuran wird die Lösung von 223 g (1 Mol) 1-(5-Methyl-1,3-dioxan-5-yl)-2-bromethan-1-on in 200 ml Tetrahydrofuran bei 25°C in 2 Stunden getropft. Nach 8stündigem Rückfluß wird der anorganische Niederschlag abfiltriert und das Filtrat zur Hälfte eingeengt. Das Gemisch wird angeimpft und über Nacht bei +3°C stehen gelassen. Der Niederschlag wird abgesaugt, mit 30 ml kaltem (+5°C) Tetrahydrofuran, dann mit 80 ml Ether und anschließend mit 100 ml n-Pentan gewaschen und getrocknet. Man erhält 184 g (87,2%) 1-(5-Methyl-1,3-dioxan-5-yl)-2-(1,2,4-triazolyl-(1))-ethan-1-on als weiße Kristalle vom Schmelzpunkt 95—97°C.

### b) Herstellung des Endproduktes

Unter Wasserabscheidung am Rückfluß wird eine Mischung aus 12,7 g (0,06 Mol) 1-(5-Methyl-1,3-dioxan-5-yl)-2-(1,2,4-triazolyl)-(1))-ethan-1-on, 10,8 g (0,06 Mol) 2,4-Dichlorbenzaldehyd, 1 g Piperidin, 0,5 g Essigsäure und 150 ml Toluol 10 Stunden gerührt. Das Gemisch wird abgekühlt, dreimal mit je 80 ml Wasser gewaschen, über Natriumsulfat getrocknet und im Vakuum eingeengt. Der Rückstand wird in 250 ml Acetonitril aufgelöst, bei 40°C mit 1 g Tierkohle entfärbt, auf 40 ml eingeengt und auf 0°C abgekühlt. Der farblose Niederschlag wird abgesaugt, mit 40 ml Ether gewaschen und getrocknet. Man erhält 18,4 g (83,3%) 1-(5-Methyl-1,3-dioxan-5-yl)-2-(1,2,4-triazolyl)-(1))-3-(2,4-dichlorphenyl)-propen-1-on vom Schmelzpunkt 133—135°C.

## Beispiel 2

Zu einer Lösung von 36,1 g (0,1 Mol) 1-(5-Methyl-1,3-dioxan-5-yl)-2-(1,2,5-triazolyl-(1))-3-(2,4-dichlorphenyl)-propen-1-on in 250 ml Methanol werden zwischen −5 und 0°C 4,5 g (0,118 Mol) Natriumborhydrid portionsweise zugegeben. Nach 14stündigem Rühren bei 22°C wird der weiße Niederschlag abgesaugt und das Filtrat im Vakuum eingeengt. Niederschlag und Rückstand werden vereinigt und 0,5 Stunden mit 3 l Methylenchlorid um 500 ml 10%iger Kalilauge bei 25°C gerührt. Die organische Schicht wird abgetrennt, dreimal mit je 100 ml Wasser gewaschen, über Natriumsulfat getrocknet und eingeengt. Der Rückstand kristallisiert nach Zugabe von 10 ml Ether. Man erhält 31,3 g (84,6%) 1-(5-Methyl-1,3-dioxan-5-yl)-2-(1,2,4-triazolyl-(1))-3-(2,4-dichlorphenyl)-propen-1-ol als farblose Kristalle vom Schmelzpunkt 190 bis 192°C.

## Beispiel 3

Eine Mischung aus 14 g (0,0378 Mol) 1-(Methyl-1,3-dioxan-5-yl)-2-(1,2,4-triazolyl)-(1))-3-(3,4-dichlorphenyl)-propen-1-ol (Beispiel 25) 1 g Imidazol und 80 g Acetanhydrid werden 8 Stunden bei 70°C gerührt und anschließend im Vakuum eingeengt. Der Rückstand wird in 300 ml Ether aufgelöst und 0,5 Stunden mit 100 ml einer 6%igen Natriumhydrogencarbonat-Lösung gerührt. Die organische Phase wird über Natriumsulfat getrocknet und im Vakuum — schließlich bis 50°C und 0,1 mbar — eingeengt. Man erhält 10,8 g (69,3% der Theorie) 1-(5-Methyl-1,3-dioxan-5-yl)-1-acetoxy-2-(1,2,4-triazolyl-(1))-3-(3,4-dichlorphenyl)-propen-2 als blaßgelbes Harz.
$^1$H-NMR (80 MHz/$CDCl_3$): = 0,6 (s, 3H), 2,08 (s, 3H), 3,2—3,4 (m, 2H), 3,8—4,0 (g, 2H), 4,4—4,9 (2dd, zus. 2H), 5,5 (s, 1H), 6,3—7,4 (m, zus. 4H), 8,0 (s, 1H) und 8,2 ppm (s, 1H).
In entsprechender Weise können die in der folgenden Tabelle aufgeführten Verbindungen hergestellt werden.

Tabelle

| Bei-spiel Nr. | Ar | $R^1$ | $R^2$ | X | Fp/°C | IR (Film) [$cm^{-1}$] |
|---|---|---|---|---|---|---|
| 4 | $C_6H_5-$ | $CH_3-$ | H | $-CO-$ | Harz | 3100, 2965, 1660, 1585, 1485, 1195, 1150, 1065, 920, 750, 690, 668 |
| 5 | α-Furyl | $CH_3-$ | H | $-CO-$ | 108–110 | |
| 6 | α-Furyl | $CH_3-$ | H | $\overset{OH}{\overset{\vert}{-CH-}}$ | 99–102 | |
| 8 | α-Naphthyl | $CH_3-$ | $C_2H_5-$ | $-CO-$ | Harz | 3112, 2970, 2850, 1668, 1490, 1380, 1265, 1155, 1080, 918, 792, 770, 665 |
| 9 | α-Naphthyl | $CH_3-$ | $C_2H_5$ | $\overset{OH}{\overset{\vert}{-CH-}}$ | Harz | 3300, 3060, 2965, 2850, 1508, 1468, 1275, 1160, 1090, 925, 880, 778, 675 |
| 10 | p-$C_6H_5-C_6H_4$ | $CH_3-$ | H | $-CO-$ | Harz | 3100, 3010, 2845, 1668, 1590, 1488, 1150, 1015, 920, 910, 830, 755, 725, 690, 665 |
| 11 | p-$C_6H_5-C_6H_4$ | $CH_3-$ | H | $\overset{OH}{\overset{\vert}{-CH-}}$ | 122–124 | |
| 12 | 2-Cl-$C_6H_4$ | $CH_3-$ | H | $-CO-$ | 86–88 | |
| 13 | 3-Cl$-C_6H_4$ | $CH_3$ | H | $-CO-$ | Harz | 3118, 3060, 2980, 2850, 1680, 1600, 1592, 1270, 1155, 1070, 920, 780, 680, 665 |
| 14 | 3-Cl$-C_6H_4$ | $CH_3-$ | H | $\overset{OH}{\overset{\vert}{-CH-}}$ | Harz | 3250, 3120, 2845, 1493, 1266, 1155, 1078, 1023, 918, 780, 668 |
| 15 | 4-Cl-$C_6H_4$ | $CH_3-$ | H | $-CO-$ | Harz | 3100, 2960, 2840, 1665, 1572, 1475, 1390, 1265, 1155, 1082, 1025, 1000, 920, 820, 752, 668 |
| 16 | desgl. | $CH_3-$ | $C_2H_5-$ | $-CO-$ | Harz | 3108, 2960, 2845, 1685, 1578, 1490, 1262, 1150, 1080, 940, 915, 815, 752, 665, 640 |

Fortsetzung

| Bei-spiel Nr. | Ar | $R^1$ | $R^2$ | X | Fp/°C | IR (Film) [$cm^{-1}$] |
|---|---|---|---|---|---|---|
| 17 | 4-Cl-$C_6H_4$ | $CH_3$ | $C_2H_5$— | OH<br>\|<br>—CH— | Harz | 3380, 3120, 2960, 2840, 1490, 1480, 1254, 1150, 1125, 1080, 915, 870, 810, 665 |
| 18 | 4-Br—$C_6H_4$ | $CH_3$— | H | —CO— | Harz | 3100, 2970, 2840, 1665, 1567, 1485, 1470, 1265, 1155, 1060, 1025, 1000 918, 815, 750, 670 |
| 19 | desgl. | $CH_3$— | H | OH<br>\|<br>—CH— | Harz | 3400, 2970, 2850, 1495, 1480, 1270, 1158, 1132, 1068, 1022, 1004, 918, 810, 668 |
| 20 | desgl. | $CH_3$— | H | O—$COCH_3$<br>\|<br>—CH— | Harz | 3310, 2950, 2840, 1730, 1490, 1475, 1360, 1218, 1150, 1020, 915, 806, 662 |
| 21 | desgl. | $CH_3$— | H | O—$COCH_2CH_3$<br>\|<br>—CH— | Harz | 3118, 2970, 2845, 1730, 1578, 1490, 1478, 1265, 1150, 1070, 1000, 918, 810, 665 |
| 22 | desgl. | $CH_3$— | $C_2H_5$— | —CO— | Harz | 3115, 2970, 2850, 1690, 1580, 1492, 1268, 1128, 1085, 1065, 1002, 920, 816, 752, 670 |
| 23 | desgl. | $CH_3$— | $C_2H_5$— | OH<br>\|<br>—CH— | Harz | 3300, 2960, 2845, 1492, 1476, 1390, 1265, 1150, 1082, 1060, 1000, 915, 870, 810, 665 |
| 24 | 3,4-DiCl−$C_6H_3$− | $CH_3$— | H | —CO— | Harz | 3110, 2975, 2855, 1680, 1600, 1475, 1462, 1270, 1200, 1155, 1070, 920, 880, 760, 665 |
| 25 | desgl. | $CH_3$— | H | OH<br>\|<br>—CH— | Harz | 3400, 2958, 2840, 1490, 1460, 1152, 1125, 1075, 1020, 914, 884, 810, 665 |

Fortsetzung

| Beispiel Nr. | Ar | $R^1$ | $R^2$ | X | Fp/°C | IR (Film) [$cm^{-1}$] |
|---|---|---|---|---|---|---|
| 26 | 3,4-DiCl$-C_6H_3-$ | $CH_3-$ | H | $-CO-$ | Harz | 3105, 2970, 2850, 1690, 1430, 1450, 1380, 1265, 1150, 1080, 1022, 920, 870, 810, 665 |
| 27 | desgl. | $CH_3-$ | $C_2H_5-$ | OH<br>\|<br>$-CH-$ | Harz | 3300, 2970, 2855, 1505, 1470, 1400, 1275, 1135, 1090, 1030, 925, 880 |
| 28 | 4-Cl$-C_6H_4$ | $C_2H_5-$ | $C_2H_5-$ | $-CO-$ | Harz | 3108, 2960, 2924, 2845, 1675, 1480, 1392, 1265, 1123, 1080, 916, 815, 775, 664 |
| 29 | 2,4-DiCl$-C_6H_3$ | $C_2H_5-$ | $C_2H_5-$ | $-CO-$ | Harz | 3100, 2964, 2930, 2850, 1680, 1576, 1640, 1375, 1266, 1125, 1095, 918, 876, 860, 826, 812, 664 |
| 30 | desgl. | $CH_3-$ | $C_2H_5-$ | $-CO-$ | Harz | 3100, 2970, 2850, 1680, 1575, 1490, 1460, 1265, 1155, 1125, 1090, 992, 918, 860, 665 |
| 31 | desgl. | $CH_3-$ | $C_2H_5-$ | OH<br>\|<br>$-CH-$ | Harz | 3300, 2970, 2845, 1580, 1495, 1460, 1265, 1152, 1085, 1045, 918 |
| 32 | desgl. | $CH_3-$ | $-CH(CH_3)_2$ | $-CO-$ | Harz | 3100, 2970, 2860, 1690, 1580, 1465, 1380, 1270, 1185, 1130, 1095, 995, 920, 860, 830, 670 |
| 33 | desgl. | $CH_3-$ | $-CH(CH_3)_2$ | OH<br>\|<br>$-CH-$ | Harz | 3300, 2960, 2845, 1580, 1500, 1465, 1268, 1130, 1095, 995, 918, 860, 815, 670 |
| 34 | desgl. | $CH_3-$ | $n-C_4H_9$ | $-OH-$ | Harz | 3120, 2950, 2860, 1710, 1685, 1578, 1496, 1462, 1380, 1268, 1130, 1097, 945, 860, 770, 680 |

Fortsetzung

| Bei-spiel Nr. | Ar | $R^1$ | $R^2$ | X | Fp/°C | IR (Film) [$cm^{-1}$] |
|---|---|---|---|---|---|---|
| 35 | 2,4-DiCl—$C_6H_3$ | $CH_3$— | N—$C_4H_9$ | OH<br>\|<br>—CH— | Harz | 3200, 2945, 2850, 1498, 1467, 1365, 1268, 1125, 1098, 1018, 990, 925, 855, 815 |
| 36 | 2,3,4-TrCl-$C_6H_2$ | $CH_3$— | H | —CO— | 110–140 | |
| 37 | desgl. | $CH_3$— | H | OH<br>\|<br>—CH— | 156–159 | |
| 38 | desgl. | $CH_3$— | H | O—$COCH_3$<br>\|<br>—CH— | 138–140 | |
| 39 | 3-$CF_3C_6H_4$ | $CH_3$— | H | —CO— | Harz | 3115, 2980, 2850, 1680, 1493, 1324, 1156, 1118, 1068, 1025, 918, 795, 695, 670, 655 |
| 40 | desgl. | $CH_3$— | H | OH<br>\|<br>—CH— | 95–98 | |
| 41 | desgl. | $CH_3$— | H | O—CO—$CH_2CH_3$<br>\|<br>—CH— | Harz | 3110, 2970, 2840, 1730, 1490, 1320, 1190, 1150, 1115, 1065, 915, 795, 690, 665, 650 |
| 42 | 4-$CF_3$—$C_6H_4$ | $CH_3$— | H | —CO— | Harz | 3110, 2980, 2845, 1680, 1605, 1490, 1315, 1155, 1115, 1056, 915, 828, 665 |
| 43 | desgl. | $CH_3$— | H | OH<br>\|<br>—CH— | 108–118 | |
| 44 | 4-$CH_3$—$C_6H_4$ | $CH_3$— | H | —CO— | 82–84 | |
| 45 | desgl. | $CH_3$— | H | OH<br>\|<br>—CH— | Harz | 3260, 3120, 2845, 1505, 1492, 1290, 1150, 1130, 1068, 1024, 930, 918, 880, 808, 668 |
| 46 | desgl. | $CH_3$— | $C_2H_5$— | —CO— | Harz | 3110, 2695, 2845, 1675, 1595, 1490, 1380, 1262, 1142, 1125, 1080, 918, 805, 666 |

Fortsetzung

| Beispiel Nr. | Ar | $R^1$ | $R^2$ | X | Fp/°C | IR (Film) [$cm^{-1}$] |
|---|---|---|---|---|---|---|
| 47 | $4-CH_3-C_6H_4$ | $CH_3-$ | $C_2H_5-$ | $-\overset{\overset{OH}{\vert}}{C}H-$ | Harz | 3300, 2960, 2840, 1500, 1455, 1390, 1265, 1154, 1128, 1084, 1022, 918, 872, 805, 668 |
| 48 | 4-tert.Butyl-$C_6H_4$ | $CH_3-$ | H | $-CO-$ | Harz | 3118, 2960, 2860, 1670, 1590, 1490, 1355, 1260, 1165, 1065, 1025, 995, 915, 825, 750, 668 |
| 49 | desgl. | $CH_3-$ | H | $-\overset{\overset{OH}{\vert}}{C}H-$ | Harz | 3300, 2955, 2850, 1492, 1450, 1352, 1260, 1155, 1072, 1025, 918, 820, 668 |
| 50 | $4-CH_3O-C_6H_4$ | $CH_3-$ | H | $-CO-$ | 132–134 | |
| 51 | desgl. | $CH_3-$ | $C_2H_5-$ | $-CO-$ | Harz | 3115, 2970, 2840, 1670, 1590, 1505, 1250, 1170, 1165, 1125, 1080, 1020, 918, 826, 668 |
| 52 | desgl. | $CH_3-$ | $C_2H_5-$ | $-\overset{\overset{OH}{\vert}}{C}H-$ | Harz | 3320, 2840, 1595, 1500, 1455, 1385, 1240, 1080, 1020, 915, 870, 820, 668 |
| 53 | $4-C_2H_3-O-C_6H_4$ | $CH_3-$ | H | $-CO-$ | 117–120 | |
| 54 | desgl. | $CH_3-$ | H | $-\overset{\overset{OH}{\vert}}{C}H-$ | 123–125 | |
| 55 | $4-O_2N-C_6H_4$ | $CH_3-$ | H | $-CO-$ | Harz | 3110, 2975, 2850, 1680, 1590, 1510, 1340, 1155, 1025, 915, 840, 742, 668 |
| 56 | desgl. | $CH_3-$ | H | $-\overset{\overset{OH}{\vert}}{C}H-$ Isomer I | 201–203 | |
| 57 | desgl. | $CH_3-$ | H | $-\overset{\overset{OH}{\vert}}{C}H-$ Isomerengemisch | Harz | 3250, 2850, 1590, 1510, 1335, 1152, 1130, 1070, 1020, 918, 842, 749, 690, 665 |

Fortsetzung

| Beispiel Nr. | Ar | $R^1$ | $R^2$ | X | Fp/°C | IR (Film) [$cm^{-1}$] |
|---|---|---|---|---|---|---|
| 58 | 4-$F_3C-C_6H_4-$ | $CH_3-$ | $C_2H_5-$ | $-CO-$ | Harz | 3110, 2965, 1680, 1488, 1312, 1155, 1115, 915, 825, 660 |
| 59 | desgl. | $CH_3-$ | $C_2H_5-$ | $-\overset{OH}{\overset{\vert}{C}}H-$<br>Isomer I | 180–182 | |
| 60 | desgl. | $CH_3-$ | $C_2H_5-$ | $-\overset{OH}{\overset{\vert}{C}}H-$<br>Isomerengemisch | Harz | 3230, 2980, 1622, 1507, 1320, 1170, 1130, 1066, 1020, 925, 883, 825, 672 |
| 61 | $C_2H_5-O-C_6H_4$ | $CH_3-$ | $C_2H_5-$ | $-CO-$ | Harz | 3115, 2980, 1670, 1590, 1500, 1383, 1250, 1174, 1130, 1084, 1035, 920, 830, 672 |
| 62 | desgl. | $CH_3-$ | $C_2H_5-$ | $-\overset{OH}{\overset{\vert}{C}}H-$ | Harz | 3300, 2970, 1600, 1500, 1240, 1182, 1085, 1038, 918, 875, 816, 668 |
| 63 | $4-O_2N-C_6H_4$ | $CH_3-$ | $C_2H_5-$ | CO | Harz | 3115, 2975, 1690, 1595, 1515, 1342, 1225, 1160, 1130, 1000, 922, 856, 820, 745 |
| 64 | desgl. | $CH_3-$ | $C_2H_5-$ | $-\overset{OH}{\overset{\vert}{C}}H-$ | Harz | 3300, 2960, 1590, 1510, 1336, 1270, 1130, 1083, 918, 873, 845, 735 |
| 65 | $C_6H_5$ | $CH_3-$ | $C_2H_5-$ | $-CO-$ | Harz | 3120, 2975, 1698, 1495, 1445, 1382, 1268, 1158, 1130, 1086, 922, 755, 695, 672 |
| 66 | $C_6H_5$ | $CH_3-$ | $C_2H_5$ | $-\overset{OH}{\overset{\vert}{C}}H-$ | Harz | 3280, 2960, 1492, 1450, 1268, 1155, 1130, 1085, 920, 874, 740, 692, 668 |

Die neuen erfindungsgemäßen Wirkstoffe greifen in den Metabolimus der Pflanzen ein und können deshalb als Wachstumsregulatoren eingesetzt werden. Sie können praktisch alle Entwicklungsstadien einer Pflanze beeinflußen.

Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanzen und von der Jahreszeit,
c) von dem Applikationsort und -verfahren (Samenbeize, Bodenbehandlung oder Blattapplikation),
d) von den geoklimatischen Faktoren, z. B. Sonnenscheindauer, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.
Als vorteilhaft für die Praxis erweist sich z. B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.
Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des »Lagerns« (des Umknickens) von Pflanzen unter ungünstigen Witterungsbedingungen vor der Ernte.
Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
Durch Anwendung von Wachstumsregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.
Mit Wachstumshemmern läßt sich beispielsweise bei Winterraps auch die Frostresistenz erheblich erhöhen. Dabei werden einerseits das Längenwachstum und die Entwicklung zu einer üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phasen neigen. Auch bei anderen Kulturen, z. B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und — wegen der relativ geringen Blatt- bzw. Pflanzenmasse — dem Befall mit verschiedenen Krankheiten (z. B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den neuen Wirkstoffen lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.
Dabei können die neuen Stoffe Ertragssteigerungen durch Eingriffe in den pflanzlichen Stoffwechsel bzw. durch Förderung oder Hemmung des vegetativen und/oder des generativen Wachstums verusachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.
Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern der Bäume wesentlich.
Die Wirkung der neuen Verbindungen ist besser als bei bekannten Wachstumsregulatoren. Die Wirkung zeigt sich sowohl bei Monokotylen, z. B. Getreide wie Weizen, Gerste, Roggen, Hafer und

Reis oder Mais oder Gräsern als auch insbesondere bei Dikotylen (z. B Sonnenblumen, Tomaten, Erdnüssen, Reben, Baumwolle, Raps und vor allem Soja) und verschiedenen Zierpflanzen wie Chrysanthemen, Poinsettien und Hibiskus.

Die erfindungsgemäßen Wirkstoffe können den Kulturpflanzen sowohl vom Samen her (als Saatgutbeizmittel) als auch über den Boden, d. h. durch die Wurzel sowie — besonders bevorzugt — durch Spritzung über das Blatt zugeführt werden.

Die erfindungsgemäßen Verbindungen können den Kulturpflanzen sowohl durch den Samen (als Saatgutbeizmittel), als auch über den Boden, d. h. durch die Wurzel sowie durch Spritzung über das Blatt zugeführt werden. Infolge der hohen Pflanzenverträglichkeit kann die Aufwandmenge stark varriiert werden.

Bei der Saatgutbehandlung werden im allgemeinen Wirkstoffmengen von 0,001 bis 50 g je Kilogramm Saatgut, vorzugsweise 0,01 bis 10 g benötigt.

Für die Blatt- und Bodenbehandlung sind im allgemeinen Gaben von 0,1 bis 12 kg/ha bevorzugt, 0,25 bis 3 kg/ha als ausreichend zu betrachten.

Die erfindungsgemäßen Mittel können in Form üblicher Formulierung angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin), Dimethylformamid und Wasser; feste Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel oder sonstige oberflächenaktive Mittel, wie nichtionogene und anionische Emulgatoren (z. B. Polyoxyethylen-Fettalkohol-Ether, Alkylsulfonate) und Dispergiermittel Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gew.-% Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I 20 Gewichtsteile der Verbindung des Beispiels 7 werden in 3 Gewichtsteilen des Natriumsalzes der Diisobutylnapthtalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Michung in 20 000 Gewichtsteilen Wasser erhält man eine Spritzbrühe, die 0,1 Gew.-% des Wirkstoffs enthält.

II 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.-% des Wirkstoffs enthält.

III 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 3 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

V 20 Teile der Verbindung des Beispiels 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI Man vermischt 90 Gewichtsteile der Verbindung des Beispiels 1 mit 10 Gewichtsteilen N-Methyl-$\alpha$-pyrrolidon und erhält eine Lösung, die zur Anwendung in Form kleinster Tropfen geeignet ist.

VII 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

VIII 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IX 10 Gewichtsteile der Verbindung des Beispiels 10, 20 Gewichtsteile Polyoxyethylensorbitanmonolaurat ([R]Tween 20), 20 Gewichtsteile Methanol und 50 Gewichtsteile Wasser werden zu einer Lösung verrührt, die 10 Gew.-% des Wirkstoffs enthält. Durch Hinzufügen von weiterem Wasser können stärker verdünnte Lösungen hergestellt werden.

Die erfindungsgemäßen Mittel können in diesen Aufwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d. h. die Wirksamkeit des Kombinationsproduktes ist größer als die addierten Wirksamkeiten der Einzelkomponenten.

Fungizide, die mit den erfindungsgemäßen Verbindungen kombiniert werden können, sind beispielsweise Dithiocarbamate und deren Derivate, wie

Ferridimethyldithiocarbamat,
Zinkdimethyldithiocarbamat,
Manganethylenbisthiocarbamat,
Mangan-Zink-ethylendiamin-bis-dithiocarbamat,
Zinkethylenbisthiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis-dithiocarbamat) und
N,N'-Polypropylen-bis-(thiocarbamat)-disulfid;

Nitrophenolderivate, wie

Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie

N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
O,O-Diethyl-phthalimidophosphonthionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2)-benzimidazol,
Piperazin-1,4-diyl-bis-1-(2,2,2-trichlor-ethyl)-formamid,
2-Thiazolyl-(4)-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie

Dodecylguanidinacetat,
3-(2-(3,5-Dimethyl-2-oxycyclohexyl)-2-hydroxyethyl)-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl(2)-alaninat,
D,L-N-(2,6-Dimethyl-phenyl)-N-(2'-methoxyacetyl)-alaninmethylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazinnatriumsulfonat,
1-Chlor-2-nitro-propan,

Polychlornitrobenzole, wie

Pentachlornitrobenzol,
Methylisocyanat,
fungizide Antibiotika, wie
Griseofulvin oder
Kasugamycin,
Tetrafluordichloraceton,
1-Phenylthiosemicarbazid,
Bordeauxmischung,
nickelhaltige Verbindungen und
Schwefel.

Gewächshausversuch

Zur Bestimmung der wachstumsregulierenden Eigenschaft der Prüfsubstanzen wurden Testpflanzen auf ausreichend mit Nährstoffen versorgtem Torfkultursubstrat in Kunststoffgefäßen von ca. 12,5 cm Durchmesser angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. In diesen Versuchen, die an Sommergerste, Rasen und Sommerraps durchgeführt wurden, zeigten insbesondere die Substanzen der Beispiele 1, 2, 4, 5, 7, 12, 15, 18, 19, 25, 33, 35 und 50 eine bessere Wirkung als die Vergleichssubstanzen Chlorcholinchlorid (CCC) und 3,3-Dimethyl-2-(1,2,4-triazolyl-(1))-1-(4-chlorbenzoyl)-butan.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

**Patentansprüche für die Vertragsstaaten: BE, CH, DE, FR, GB, IT, LI, LU, NL, SE**

1. 1,3-Dioxan-5-yl-alkenyltriazole der Formel I

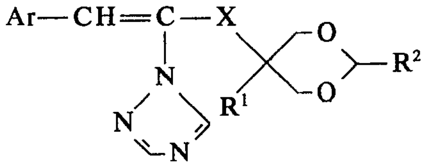

(I)

in der

$R^1$ und $R^2$ gleich oder verschieden sind und Wasserstoff oder Alkyl mit 1 bis 5 C-Atomen bedeuten,
Ar Furanyl, Thienyl, Biphenylyl, Naphthyl oder Phenyl bedeutet, wobei der Phenylrest durch Alkyl,

Alkoxy oder Alkenyl mit jeweils 1 bis 5 C-Atomen, Fluor, Chlor, Brom, Jod, Nitro, Trifluormethyl und ferner durch Phenoxy substituiert sein kann und

X eine C=O, –CH(OH)– oder –CH(O–COR³)-Gruppe bedeutet, wobei $R^3$ einen gegebenenfalls durch Halogen oder Alkoxy substituierten Alkyl mit 1 bis 5 C-Atomen oder Alkenyl mit 2 bis 5 C-Atomen oder Aryl bedeutet

sowie deren Salze und Metallkomplexe.

2. Mittel zur Regulierung des Pflanzenwachstums, enthaltend eine oder mehrere Verbindungen gemäß Anspruch 1.

3. Mittel zur Regulierung des Pflanzenwachstums, enthaltend eine oder mehrere Verbindungen gemäß Anspruch 1 und einen flüssigen oder festen Trägerstoff sowie gegebenenfalls ein oder mehrere oberflächenaktive Mittel.

4. Verwendung von Verbindungen gemäß Anspruch 1 zur Regulierung des Pflanzenwachstums.

5. Verfahren zur Regulierung des Pflanzenwachstums, dadurch gekennzeichnet, daß man eine oder mehrere Verbindungen gemäß Anspruch 1 auf Pflanzen oder deren Lebensraum einwirken läßt.

6. Verfahren zur Herstellung von das Pflanzenwachstum regulierenden Mitteln, dadurch gekennzeichnet, daß man eine oder mehrere Verbindungen gemäß Anspruch 1 mit festen oder flüssigen Trägerstoffen sowie gegebenenfalls einem oder mehreren oberflächenaktiven Mitteln mischt.

7. Verfahren zur Herstellung von 1,3-Dioxan-5-yl-alkenyltriazole der Formel I

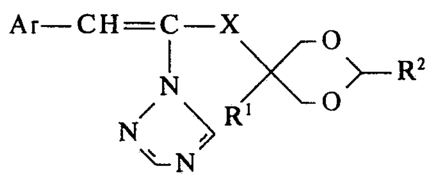

(I)

in der

$R^1$ und $R^2$ gleich oder verschieden sind und Wasserstoff oder Alkyl mit 1 bis 5 C-Atomen bedeuten,

Ar Furanyl, Thienyl, Biphenylyl, Naphthyl oder Phenyl bedeutet, wobei der Phenylrest durch Alkyl, Alkoxy oder Alkenyl mit jeweils 1 bis 5 C-Atomen, Fluor, Chlor, Brom, Jod, Nitro, Trifluormethyl und ferner durch Phenoxy substituiert sein kann und

X eine C=O, –CH(OH)– oder –CH(O–COR³)-Gruppe bedeutet, wobei $R^3$ einen gegebenenfalls durch Halogen oder Alkoxy substituierten Alkyl mit 1 bis 5 C-Atomen oder Alkenyl mit 2 bis 5 C-Atomen oder Aryl bedeutet

sowie deren Salze und Metallkomplexen, dadurch gekennzeichnet, daß man einen Aldehyd der Formel II

$$Ar - CH = O \qquad (II)$$

in der Ar die oben genannte Bedeutung hat, mit einem 1-(1,3-Dioxan-5-yl)-2-(1,2,4-triazolyl-(1))-ethan-1-on-Derivat der Formel III

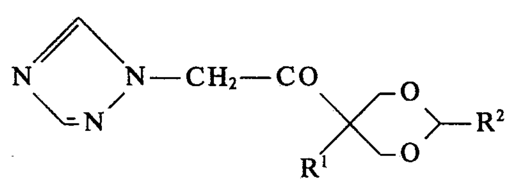

(III)

in welcher $R^1$ und $R^2$ die oben angegebenen Bedeutungen haben, kondensiert, ggf. die so erhaltenen Verbindungen zum Alkohol reduziert, ggf. die reduzierte Verbindung mit einem Säurehalogenid der Formel IV

$$Y - CO - R^3 \qquad (IV)$$

in der $R^3$ dasselbe wie oben bedeutet und Y Chlor oder Brom bedeutet, oder mit einem Säureanhydrid der Formel V

$$(R^3 - CO)_2O \qquad (V)$$

umsetzt und gegebenenfalls die erhaltenen Verbindungen in ihre Salze oder Metallkomplexe überführt.

**Patentansprüche für den Vertragsstaat: AT**

1. Verfahren zur Herstellung von 1,3-Dioxan-5-yl-alkenyltriazole der Formel I

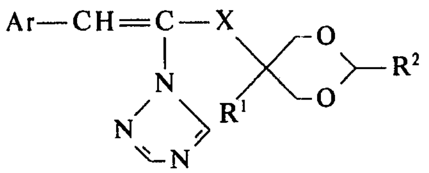

(I)

in der

$R^1$ und $R^2$ gleich oder verschieden sind und Wasserstoff oder Alkyl mit 1 bis 5 C-Atomen bedeuten,
Ar Furanyl, Thienyl, Biphenylyl, Naphthyl oder Phenyl bedeutet, wobei der Phenylrest durch Alkyl, Alkoxy oder Alkenyl mit jeweils 1 bis 5 C-Atomen, Fluor, Chlor, Brom, Jod, Nitro, Trifluormethyl und ferner durch Phenoxy substituiert sein kann und
X eine C=O, $-CH(OH)-$ oder $-CH(O-COR^3)$-Gruppe bedeutet, wobei $R^3$ einen gegebenenfalls durch Halogen oder Alkoxy substituierten Alkyl mit 1 bis 5 C-Atomen oder Alkenyl mit 2 bis 5 C-Atomen oder Aryl bedeutet

sowie deren Salze und Metallkomplexen, dadurch gekennzeichnet, daß man einen Aldehyd der Formel II

$$Ar - CH = O \qquad (II)$$

in der Ar die oben genannte Bedeutung hat, mit einem 1-(1,3-Dioxan-5-yl)-2-(1,2,4-triazolyl-(1))-ethan-1-on-Derivat der Formel III

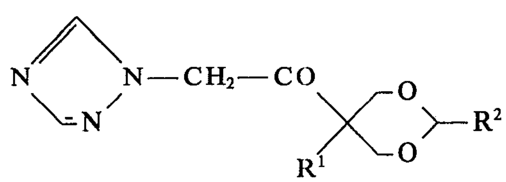

(III)

in welcher $R^1$ und $R^2$ die oben angegebenen Bedeutungen haben, kondensiert, ggf. die so erhaltenen Verbindungen zum Alkohol reduziert, ggf. die reduzierte Verbindung mit einem Säurehalogenid der Formel IV

$$Y - CO - R^3 \qquad (IV)$$

in der $R^3$ dasselbe wie oben bedeutet und Y Chlor oder Brom bedeutet, oder mit einem Säureanhydrid der Formel V

$$(R^3 - CO)_2O \qquad (V)$$

umsetzt und gegebenenfalls die erhaltenen Verbindungen in ihre Salze oder Metallkomplexe überführt.

2. Mittel zur Regulierung des Pflanzenwachstums, enthaltend eine oder mehrere Verbindungen gemäß Anspruch 1.

3. Verwendung von Verbindungen gemäß Anspruch 1 zur Regulierung des Pflanzenwachstums.

4. Verfahren zur Regulierung des Pflanzenwachstums, dadurch gekennzeichnet, daß man eine oder mehrere Verbindungen gemäß Anspruch 1 auf Pflanzen oder deren Lebensraum einwirken läßt.

**Claims for the Contracting States: BE, CH, DE, FR, GB, IT, LI, LU, NL, SE**

1. A 1,3-dioxan-5-yl-alkenyltriazole of the formula I

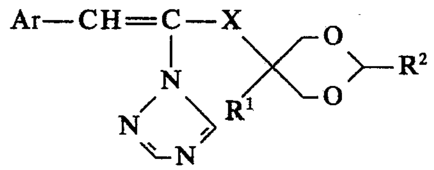

(I)

where $R^1$ and $R^2$ are identical or different and each is hydrogen or alkyl of 1 to 5 carbon atoms, Ar is furanyl, thienyl, biphenylyl or naphthyl, or is phenyl which is unsubstituted or substituted by alkyl, alkoxy or alkenyl, each of 1 to 5 carbon atoms, or by fluorine, chlorine, bromine, iodine, nitro, trifluoromethyl or phenoxy, and X is C=O, –CH(OH)– or –CH(O–COR$^3$)-, where $R^3$ is unsubstituted or halogen- or alkoxy-substituted alkyl of 1 to 5 carbon atoms or alkenyl of 2 to 5 carbon atoms or aryl, and its salts and metal complexes.

2. An agent for regulating plant growth, containing one or more compounds as claimed in claim 1.

3. An agent for regulating plant growth, containing one or more compounds as claimed in claim 1, a solid or liquid carrier and, if desired, one or more surfactants.

4. The use of a compound as claimed in claim 1 for regulating plant growth.

5. A process for regulating plant growth, wherein one or more compounds as claimed in claim 1 are allowed to act on the plants or their habitat.

6. A process for producing plant growth-regulating agents, wherein one or more compounds as claimed in claim 1 are mixed with solid or liquid carriers and, if desired, with one or more surfactants.

7. A process for preparing a 1,3-dioxan-5-yl-alkenyl-triazole of the formula I

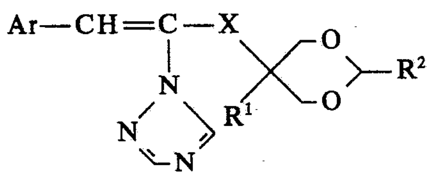

(I)

where $R^1$ and $R^2$ are identical or different and each is hydrogen or alkyl of 1 to 5 carbon atoms, Ar is furanyl, thienyl, biphenylyl or naphthyl, or is phenyl which is unsubstituted or substituted by alkyl, alkoxy or alkenyl, each of 1 to 5 carbon atoms, or by fluorine, chlorine, bromine, iodine, nitro, trifluoromethyl or phenoxy, and X is C=O, –CH(OH)– or –CH(O–COR$^3$)-, where $R^3$ is unsubstituted or halogen- or alkoxy-substituted alkyl of 1 to 5 carbon atoms or alkenyl of 2 to 5 carbon atoms or aryl, and its salts and metal complexes, wherein an aldehyde of the formula II

$$Ar - CH = O \quad (II)$$

where Ar has the above meanings, is condensed with a 1-(1,3-dioxan-5-yl)-2-(1,2,4-triazol-1-yl)-ethan-1-one derivate of the formula III

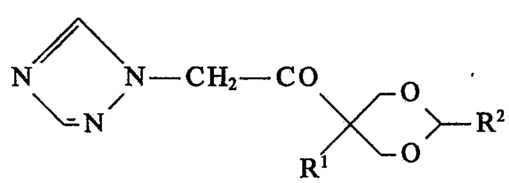

(III)

where $R^1$ and $R^2$ have the above meanings, and, if desired, the resulting compound is reduced to the alcohol and then, if desired, the reduced compound is reacted with an acid halide of the formula IV

$$Y - CO - R^3 \quad (IV)$$

where $R^3$ has the above meanings and Y is chlorine or bromine, or with an acid anhydride of the formula V

$$(R^3 - CO)_2O \quad (V)$$

and, if appropriate, the compound obtained is converted into its salts or metal complexes.

**Claims for the Contracting State: AT**

1. A process for preparing a 1,3-dioxan-5-yl-alkenyltriazole of the formula I

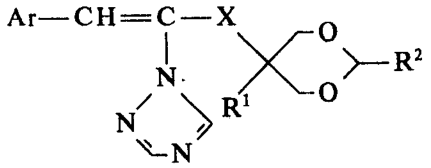

(I)

where $R^1$ and $R^2$ are identical or different and each is hydrogen or alkyl of 1 to 5 carbon atoms, Ar is furanyl, thienyl, biphenylyl or naphthyl, or is phenyl which is unsubstituted or substituted by alkyl, alkoxy or alkenyl, each of 1 to 5 carbon atoms, or by fluorine, chlorine, bromine, iodine, nitro, trifluoromethyl or phenoxy, and X is C=O, –CH(OH)– or –CH(O–COR$^3$)-, where $R^3$ is unsubstituted or halogen- or alkoxy-substituted alkyl of 1 to 5 carbon atoms or alkenyl of 2 to 5 carbon atoms or aryl, and its salts and metal complexes, wherein an aldehyde of the formula II

$$Ar - CH = O \qquad (II)$$

where Ar has the above meanings, is condensed with a 1-(1,3-dioxan-5-yl)-2-(1,2,4-triazol-1-yl)-ethan-1-one derivative of the formula III

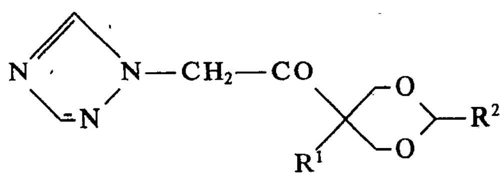

(III)

where $R^1$ and $R^2$ have the above meanings, and, if desired, the resulting compound is reduced to the alcohol and then, if desired, the reduced compound is reacted with an acid halide of the formula IV

$$Y - CO - R^3 \qquad (IV)$$

where $R^3$ has the above meanings and Y is chlorine or bromine, or with an acid anhydride of the formula V

$$(R^3 - CO)_2O \qquad (V)$$

and, if appropriate, the compound obtained is converted into its salts or metal complexes.

2. An agent for regulating plant growth, containing one or more compounds as defined in claim 1.

3. The use of a compound as defined in claim 1 for regulating plant growth.

4. A process for regulating plant growth, wherein one or more compounds as defined in claim 1 are allowed to act on the plants or their habitat.

**Revendications pour les Etats contractants: BE, CH, DE, FR, GB, IT, LI, LU, NL, SE**

1. 1,3-Dioxanne-5-yl-alcényl-triazoles de la formule

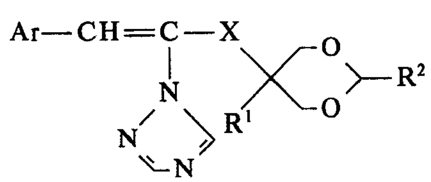

(I)

dans laquelle

$R^1$ et $R^2$, qui peuvent être identiques ou différents, désignent chacun un atome d'hydrogène ou un radical alkyle en $C_1$ à $C_5$,

Ar représente un groupe furannyle, thiényle, biphénylyle, naphtyle ou phényle ou un groupe phényle

alkyl- ou alcoxy- ou alcényl (en $C_1$ à $C_5$), fluoro-, chloro-, bromo-, iodo-, nitro-, trifluorométhyl- ou phénoxy-substitué et

X désigne un groupe $-C{=}O$, $-CH(OH)$ ou $-CH(O-COR^3)$, $R^3$ étant un radical alkyle en $C_1$ à $C_5$ éventuellement halo- ou alcoxy-substitué ou un radical alcényle en $C_2$ à $C_5$ ou un groupe aryle,

ainsi que leurs sels et complexes de métaux.

2. Composition pour la régulation de la croissance de plantes, contenant un ou plusieurs composés selon la revendication 1.

3. Composition pour la régulation de la croissance de plantes, contenant un ou plusieurs composés selon la revendictation 1, une matière support liquide ou solide et éventuellement une ou plusieurs substances tensioactives.

4. Utilisation de composés selon la revendication 1 pour la régulation de la croissance de plantes.

5. Procédé pour la croissance de plantes, caractérisé en ce que l'on fait agir un ou plusieurs composés selon la revendication 1 sur les plantes ou sur leur biotope.

6. Procédé de préparation de compositions pour la régulation de la croissance de plantes, caractérisé en ce que l'on mélange un ou plusieurs composés selon la revendication 1 avec des matières supports liquides ou solides et éventuellement une ou plusieurs substances tensioactives.

7. Procédé de préparation de 1,3-dioxanne-5-yl-alcényltriazoles de la formule I·

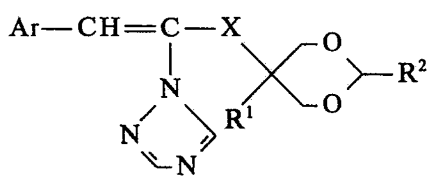

(I)

dans laquelle

$R^1$ et $R^2$, qui peuvent être identiques ou différents, désignent chacun un atome d'hydrogène ou un radical alkyle en $C_1$ à $C_5$,

Ar représente un groupe furannyle, thiényle, biphénylyle, naphtyle ou phényle ou un groupe phényle alkyl- ou alcoxy- ou alcényl- (en $C_1$ à $C_5$), fluoro-, chloro-, bromo-, iodo-, nitro-, trifluorométhyl- ou phénoxy-substitué et

X désigne un groupe $-C{=}O$, $-CH(OH)$ ou $-CH(O-COR^3)$, $R^3$ étant un radical alkyle en $C_1$ à $C_5$ éventuellement halo- ou alcoxy-substitué ou un radical alcényle en $C_2$ à $C_5$ ou un groupe aryle,

ainsi qui de leurs sels et complexes de métaux, caractérisé en ce que l'on condense un aldéhyde de la formule II

$$Ar - CH = O \qquad (II)$$

dans laquelle Ar possède la signification définie, et un dérivé de la 1-(1,3-dioxanne-5-yl)-2-(1,2,4-triazolyl-(1))-éthane-1-one de la formule III

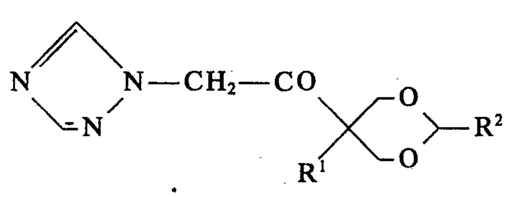

(III)

dans laquelle $R^1$ et $R^2$ possèdent les significations définies, on réduit éventuellement le composé ainsi obtenu en alcool et on fait éventuellement réagir le composé réduit avec un halogénure d'acide de la formule IV

$$Y - CO - R^3 \qquad (IV)$$

dans laquelle $R^3$ possède la signification définie et Y désigne du chlore ou du brome, ou avec un anhydride d'acide de la formule V

$$(R^3 - CO)_2O \qquad (V)$$

et l'on transforme éventuellement les composés obtenus en leurs sels ou complexes de métaux.

**Revendications pour l'Etat contractant: AT**

1. Procédé de préparation de 1,3-dioxanne-5-yl-alcényltriazoles de la formule I

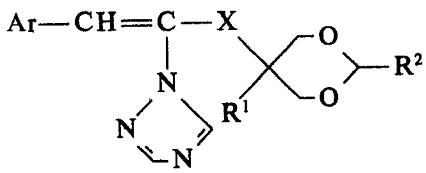

(I)

dans laquelle

$R^1$ et $R^2$, qui peuvent être identiques ou différents, désignent chacun un atome d'hydrogène ou un radical alkyle en $C_1$ à $C_5$,
Ar représente un groupe furannyle, thiényle, biphénylyle, naphtyle ou phényle ou un groupe phényle alkyl- ou alcoxy- ou alcényl- (en $C_1$ à $C_5$), fluoro-, chloro-, bromo-, iodo-, nitro-, trifluorométhyl- ou phénoxy-substitué et
X désigne un groupe $-C{=}O$, $-CH(OH)$ ou $-CH(O-COR^3)$, $R^3$ étant un radical alkyle en $C_1$ à $C_5$ éventuellement halo- ou alcoxy-substitué ou un radical alcényle en $C_2$ à $C_5$ ou un groupe aryle,

ainsi que de leurs sels et complexes de métaux, caractérisé en ce que l'on condense un aldéhyde de la formule II

$$Ar - CH = O \quad (II)$$

dans laquelle Ar possède la signification définie, et un dérivé de la 1-(1,3-dioxanne-5-yl)-2-(1,2,4-triazolyl-(1))-éthane-1-one de la formule III

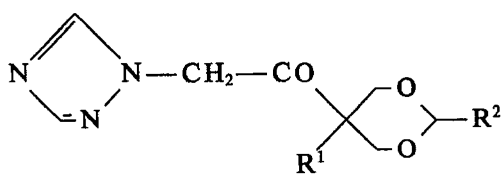

(III)

dans laquelle $R^1$ et $R^2$ possèdent les significations définies, on réduit éventuellement le composé ainsi obtenu en alcool et on fait éventuellement réagir le composé réduit avec un halogénure d'acide de la formule IV

$$Y - CO - R^3 \quad (IV)$$

dans laquelle $R^3$ possède la signification définie et Y désigne du chlore ou du brome, ou avec un anhydride d'acide de la formule V

$$(R^3 - CO)_2O \quad (V)$$

et l'on transforme éventuellement les composés obtenus en leurs sels ou complexes de métaux.

2. Composition pour la régulation de la croissance de plantes, contenant un ou plusieurs composés selon la revendication 1.

3. Utilisation de composés selon la revendication 1 pour la régulation de la croissance de plantes.

4. Procédé pour la régulation de la croissance de plantes, caractérisé en ce que l'on fait agir un ou plusieurs composés selon la revendication 1 sur les plantes ou sur leur biotope.